# FASCICULE DE BREVET EUROPEEN

(11) **EP 0 655 445 B1**
(45) Date de publication et mention de la délivrance du brevet: **27.08.1997**
(21) Numéro de dépôt: 94402642.6
(22) Date de dépôt: 21.11.1994
(51) Int. Cl.: C07D 271/10, C07D 413/12, A61K 31/41

(54) **Dérivés de 1,3,4-oxadiazol-2(3H)-one, leur préparation et leur application comme inhibiteurs de monoamine oxydase**
1,3,4-Oxadiazol-2(3H)-one-Derivate, ihre Herstellung und ihre Verwendung als Monoaminoxidase-Hemmer
1,3,4-oxadiazol-2(3H)-one Derivates, their preparation and their use as monoamine oxidase inhibitors

(30) Priorité: 26.11.1993 FR 9314151
(43) Date de publication de la demande: 31.05.1995
(73) Titulaire: SYNTHELABO, 92350 Le Plessis Robinson (FR)
(72) Inventeur: Koenig, Jean-Jacques, F-78600 Maisons Laffitte (FR); Jegham, Samir, F-95100 Argenteuil (FR); Puech, Frédéric, F-92500 Rueil Malmaison (FR); Burnier, Philippe, F-78600 Maisons Laffitte (FR)
(74) Mandataire: Thouret-Lemaitre, Elisabeth

(56) Documents cités:
- WO-A-91/08201
- EUROPEAN JOURNAL OF MEDICINAL CHEMISTRY. CHIMICA THERAPEUTICA, vol.25, 1990, PARIS FR pages 659 - 671 F. MAZOUZ ET AL. '5-Aryl-1,3,4-oxadiazol-2(3H)-one derivatives and sulfur analogues as new selective and competitive monoamine oxidase type B inhibitors'
- JOURNAL OF MEDICINAL CHEMISTRY, vol.36, no.9, 30 Avril 1993, WASHINGTON US pages 1157 - 1167 F. MAZOUZ ET AL. '5-[4-(Benzyloxy)phenyl]- 1,3,4-oxadiazol-2(3H)-one derivatives and related analogues: new reversible, highly potent, and selective monoamine oxidase type B inhibitors *'
- JOURNAL OF HETEROCYCLIC CHEMISTRY vol. 29, no. 5, 1992, PROVOH US pages 1081 - 1084 R. MILCENT ET AL. "Cyclic transformations of 5-aryl (or benzyl)-3-(2-bromoethyl-1,3,4-oxadiazol-2(3 H)-ones into 1-aminoimidazolidin-2-one and 5,6-dihydro-4H-1,3,4-oxadiazine derivatives"
- JOURNAL OF HETEROCYCLIC CHEMISTRY vol. 30, no. 4, 1993, PROVOH US pages 905 - 908 R. MILCENT ET AL. "Synthesis of 3-acylamino-2-oxazolidinone derivatives through cyclic transformation of 5-aryl (or benzyl)-1,3,4-oxadiazol-2(*H)-one derivatives

## Description

L'invention a pour objet des dérivés de 1,3,4-oxadiazol-2(3H)-one, leur préparation et leur application en thérapeutique.

Des dérivés d'oxadiazolone ont été décrits dans l'article Eur. J.Med.Chem.25(1990) page 659-671.

Leur activité sur la monoamine oxydase de type A et de type B a été déterminée.

Les composés de l'invention répondent à la formule générale (I) 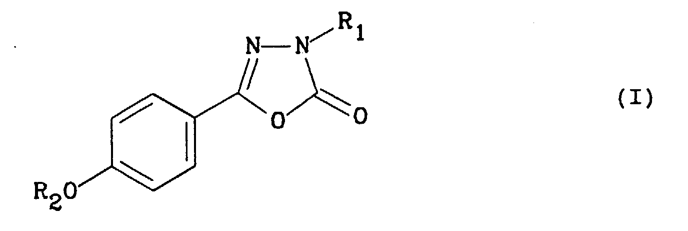 dans laquelle
R₁ représente soit un groupe (C₁₋₄)alkyle linéaire ou ramifié, substitué par un groupe hydroxy, phénoxy, (C₁₋-₄) alcoxy, (C₁₋₄) alkylthio, mercapto, (C₁₋₄)alcoxy-(C₁₋₄)alcoxy, di(C₁₋₄)alkylamino ou N-(C₁₋₄)alkyl-N-propynylamino, soit un groupe (C₃₋₄)alcynyle, et
R₂ représente soit un groupe (C₁₋₈)alkyle linéaire ou ramifié, substitué par un ou plusieurs atomes d'halogène et/ou un groupe hydroxy, 1-imidazolyle ou 3-tétrahydropyranyle, soit un groupe trifluoro(C₃₋₅)alcényle.

Les composés de formule (I) forment avec les acides pharmaceutiquement acceptables des sels d'addition qui font partie de l'invention.

Certains composés de formule (I) comportent un atome de carbone asymétrique et peuvent donc exister sous forme d'énantiomères purs ou de mélanges d'énantiomères qui font également partie de l'invention.

Les composés de formule (I) sont préparés, soit par réaction d'un composé de formule (II) 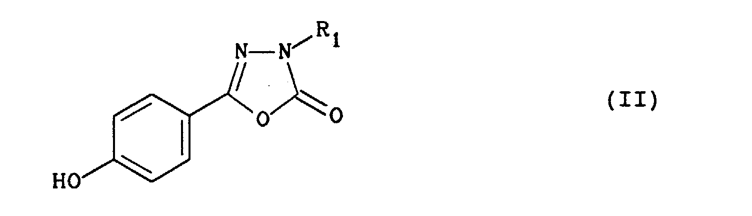 dans laquelle R₁ est défini comme ci-dessus, avec un composé de formule R₂X, dans laquelle R₂ est défini comme ci-dessus et X représente un atome d'halogène ou le groupe mésyloxy ou tosyloxy, en présence d'une base telle que le carbonate de potassium ou l'hydrure de sodium, dans un solvant tel que l'acétonitrile ou le diméthylformamide,
soit par réaction d'un composé de formule (III) 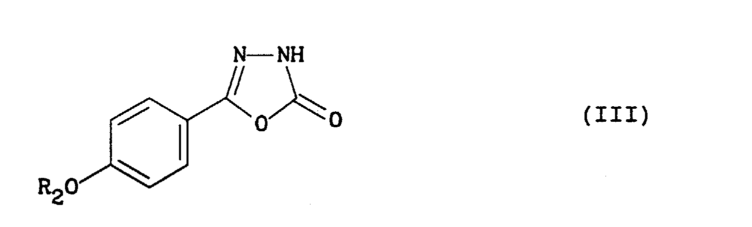 dans laquelle R₂ est défini comme ci-dessus, avec un composé de formule R₁X, dans laquelle R₁ et X sont définis comme ci-dessus, en présence d'une base telle que le carbonate de potassium ou l'hydrure de sodium, dans un solvant tel que le diméthylformamide.

Les composés de formules (II) et (III) sont préparés selon des méthodes représentées en annexe, analogues à celles qui sont décrites dans la demande de brevet WO-A-91/08201.

La méthode de préparation des composés de formule (II) consiste à protéger le groupe hydroxy d'un ester de l'acide p-hydroxybenzoïque par un groupe protecteur tel que le groupe benzyle, à faire réagir le composé de formule (VII) ainsi obtenu avec l'hydrazine pour obtenir l'hydrazide correspondant de formule (VI), qui est traité par un dérivé d'acide carbonique, tel que le phosgène, le carbonyldiimidazole ou le bis(trichlorométhyl)carbonate, pour donner un composé de formule (V), puis par un composé de formule R₁X, dans laquelle R₁ et X sont définis comme précédemment, pour donner le composé de formule (IV), qui est ensuite déprotégé.

Le composé de formule (V) dans laquelle le groupe protecteur est le groupe benzyle est un composé connu dont la préparation est décrite dans J. Med. Chem. 36, n°9, 1157-1167 (1993).

La méthode de préparation des composés de formule (III) consiste à traiter un ester de l'acide p-hydroxybenzoïque par un composé de formule R₂X, dans laquelle R₂ et X sont définis comme précédemment, à faire réagir le composé de formule (IX) ainsi obtenu avec l'hydrazine, puis à traiter l'hydrazide de formule (VIII) par un dérivé d'acide carbonique, tel que le phosgène, le carbonyldiimidazole ou le bis(trichlorométhyl)carbonate.

Les exemples suivants illustrent la présente invention. Les analyses confirment la structure des composés de l'invention.

### Exemple préparatif 1: 5-[4-(4,4,4-trifluorobutoxy)phényl]-1,3,4-oxadiazol-2(3H)-one.

### 1.1 Mésylate de 4,4,4-trifluorobutyle

Dans un tricol de 250 ml, on introduit 16,8 g de 4,4,4-trifluoro-1-butanol et 70 ml de dichlorométhane. On refroidit la solution à 0°C puis on ajoute 14,6 g de triéthylamine et 15,8 g de chlorure de mésyle. On agite le mélange pendant 30 mn en laissant revenir à la température ambiante puis on ajoute 100 ml d'eau et on décante la phase organique. On extrait la phase aqueuse avec 2 fois 20 ml de dichlorométhane puis on réunit les phases organiques, on sèche sur sulfate de sodium, on filtre, on évapore sous vide à 40°C et on distille au four à boules. On obtient 20,8 g de produit.
Point d'ébullition : 155°C sous 1 mm de Hg.

### 1.2 4-(4,4,4-Trifluorobutoxy)benzoate d'éthyle

Dans un tricol de 50 ml, on introduit 0,77 g d'hydrure de sodium en dispersion à 50% dans l'huile et 10 ml de diméthylformamide et on verse goutte à goutte, sur la suspension ainsi obtenue, une solution de 2,67 g de 4-hydroxybenzoate d'éthyle dans 20 ml de diméthylformamide. A la fin du dégagement gazeux, on ajoute 3,32 g de mésylate de 4,4,4-trifluorobutyle en solution dans 10 ml de diméthylformamide, puis on agite le mélange pendant 24 h à la température ambiante et on le verse dans 300 ml d'eau. On extrait la phase aqueuse par 3 fois 150 ml d'acétate d'éthyle, on réunit les phases organiques, on les lave avec 150 ml d'une solution aqueuse d'hydroxyde de sodium 1N puis avec 150 ml d'eau saturée en chlorure de sodium et on les évapore sous vide. On obtient 4,40 g de produit.
Point de fusion : 60,7°C.

### 1.3 Hydrazide de l'acide 4-(4,4,4-trifluorobutoxy)benzoïque

Dans un tricol de 50 ml, on introduit sous argon, à la température ambiante, 4,40 g de 4-(4,4,4-trifluorobutoxy)benzoate d'éthyle en solution dans 26 ml d'éthanol absolu. On ajoute 19,3 ml d'hydrate d'hydrazine et on chauffe le milieu réactionnel au reflux pendant 20 h. On refroidit ensuite le mélange dans un bain de glace, on filtre le précipité formé et on le sèche sous vide. On obtient 4,09 g de produit.
Point de fusion : 130°C.

### 1.4 5-[4-(4,4,4-Trifluorobutoxy)phényl]-1,3,4-oxadiazol-2(3H)-one

Dans un tricol de 100 ml, on introduit sous azote, à la température ambiante, 4,09 g d'hydrazide de l'acide 4-(4,4,4-trifluorobutoxy)benzoïque que l'on dissout à chaud dans 56 ml de toluène. On ajoute ensuite, goutte à goutte, 12,1 ml de phosgène en solution (1,93 M) dans du toluène. On chauffe le mélange à 100-110°C pendant 1 h 30 mn puis on entraîne l'excès de phosgène par un courant d'azote. On refroidit le milieu réactionnel, on filtre le précipité formé et on le sèche sous vide. On obtient 4,4 g de produit.
Point de fusion : 187°C.

### Exemple 2 : 5-[4-(4,4,4-trifluorobutoxy)phényl]-3-(2-méthoxy éthyl)-1,3,4-oxadiazol-2(3H)-one

Dans un tricol de 25 ml, on introduit 1,8 g de 5-[4-(4,4,4-trifluorobutoxy)phényl]-1,3,4-oxadiazol-2(3H)-one, 0,77 g de 2-chloroéthylméthyléther et une solution de 2,15 g de carbonate de potassium dans 20 ml de diméthylformamide. On chauffe le mélange à 100°C pendant 1 h 45 mn puis on le refroidit et on le verse dans 100 ml d'eau. On essore le précipité formé, on le lave avec 3 fois 50 ml d'eau puis on le dissout dans de l'acétate d'éthyle. On sèche la solution sur sulfate de sodium, on la filtre et on évapore le solvant. On obtient 1,8 g de produit qui est recristallisé dans 10 ml d'éthanol à 96 %. On obtient finalement 1,5 g de produit.
Point de fusion : 105,6°C.

### Exemple 3 : 5-[4-(tétrahydropyran-3-ylméthoxy)phényl]-3-(2-méthoxyéthyl)-1,3,4-oxadiazol-2(3H)-one

### 3.1 5-(4-Benzyloxyphényl)-3-(2-méthoxyéthyl)-1,3,4-oxadiazol-2(3H)-one

A une suspension de 15,2 g d'hydrure de sodium (à 50 % dans l'huile) dans 30 ml de diméthylformamide, on ajoute goutte à goutte une solution de 70,9 g de 5-(4-benzyloxyphényl)-1,3,4-oxadiazol-2(3H)-one dans 600 ml de diméthylformamide. La température croît jusqu'à 40°C. On agite le mélange pendant 30 mn, puis on verse goutte à goutte une solution de 26,5 ml de 2-chloroéthylméthyléther dans 60 ml de diméthylformamide. On chauffe à 100°C et on maintient à cette température pendant une nuit, puis on évapore une partie du diméthylformamide, on filtre, on verse le filtrat dans de l'eau et on extrait avec de l'acétate d'éthyle. On évapore ensuite la phase acétate d'éthyle et on reprend l'huile résiduelle dans du butanol à chaud. Après refroidissement, on récupère 71 g de produit.
Point de fusion : 107°C.

### 3.2 5-(4-Hydroxyphényl)-3-(2-méthoxyéthyl)-1,3,4-oxadiazol-2(3H)-one

On hydrogène sous pression réduite, en 4 heures, 71 g de 5-(4-benzyloxyphényl)-3-(2-méthoxyéthyl)-1,3,4-oxadiazol-2(3H)-one dans un mélange de 700 ml de tétrahydrofuranne et de 300 ml de méthanol, en présence de 8 g de charbon palladié à 10 % contenant 50 % d'eau et de 5 ml d'éthanol chlorhydrique. On filtre ensuite le mélange réactionnel sur silice et on concentre le filtrat sous pression réduite. Au résidu, on ajoute du toluène, puis on évapore à nouveau. On triture le produit obtenu dans de l'éther, on le filtre et on le sèche. On obtient 50,7 g de produit.
Point de fusion : 123°C.

### 3.3 3-Hydroxyméthyltétrahydropyrane

On dissout 5 g de 3-formyltétrahydropyrane (préparé selon le procédé décrit dans le brevet U.S. 5 149 821) dans un mélange 60/4 de dichlorométhane et de méthanol, puis on refroidit à une température comprise entre 0 et - 5°C et on ajoute en 8 fois, 0,831 g de borohydrure de sodium. A la fin de l'addition, on laisse remonter la température jusqu'à 10°C et on laisse à cette température pendant 40 mn.
On refroidit ensuite le mélange à 0°C, puis on ajoute goutte à goutte un mélange de 20 ml d'eau et de 2 ml d'acide chlorhydrique. La température croît jusqu'à 10°C. On décante, on extrait la phase aqueuse avec 6 fois 25 ml de dichlorométhane, on sèche sur sulfate de sodium, on filtre et on évapore le solvant. On obtient 4,13 g de produit.
On réextrait la phase aqueuse saturée en chlorure de sodium avec 3 fois 30 ml de dichlorométhane. On obtient 0,270 g de produit supplémentaires, soit 4,4 g de produit au total.

### 3.4 Mésylate de 3-tétrahydropyranylméthyle

On dissout 4 g de 3-hydroxyméthyltétrahydropyrane dans 10 ml de dichlorométhane, puis on ajoute 4,12 g de triéthylamine, on refroidit le mélange à une température comprise entre 0 et - 5°C et on ajoute goutte à goutte un mélange de 2,9 ml de chlorure de mésyle et de 100 ml de dichlorométhane. A la fin de l'addition, on laisse remonter la température jusqu'à température ambiante et on laisse à cette température pendant 45 mn.
On lave ensuite la phase organique à l'eau jusqu'à neutralité, on la sèche sur sulfate de sodium, on la filtre et on évapore le solvant. On obtient 5,9 g de produit sous forme d'huile.

### 3.5 5-[4-(Tétrahydropyran-3-ylméthoxy)phényl]-3-(2-méthoxyéthyl)-1,3,4-oxadiazol-2(3H)-one

On dissout 2 g de 5-(4-hydroxyphényl)-3-(2-méthoxyéthyl)-1,3,4-oxadiazol-2(3H)-one dans de l'acétonitrile, puis on ajoute 2,92 g de carbonate de potassium et une solution de 3,12 g de mésylate de 3-tétrahydropyranylméthyle dans de l'acétonitrile. On chauffe le mélange au reflux pendant 5 h, puis on filtre, on dilue avec de l'acétate d'éthyle et on lave avec trois fois 50 ml d'hydroxyde de sodium 2N. La phase organique est ensuite lavée à l'eau jusqu'à neutralité, séchée sur sulfate de sodium et filtrée puis le solvant est évaporé. Après deux recristallisations dans de l'éther diisopropylique, on obtient 0,7 g de produit.
Point de fusion : 77°C.

### Exemple 4 : 5-[4-(4,4,4-trifluorobutoxy)phényl]-3-(2-hydroxyéthyl)-1,3,4-oxadiazol-2(3H)-one

A une solution de 0,70 g de 5-[4-(4,4,4-trifluorobutoxy)phényl]-3-(2-méthoxyéthyl)-1,3,4-oxadiazol-2(3H)-one dans 10 ml de dichlorométhane, on ajoute goutte à goutte 1,2 g de tribromure de bore. On laisse réagir le mélange pendant une nuit puis on le traite avec une solution aqueuse de bicarbonate de sodium et on extrait au dichlorométhane. On lave la phase organique à l'eau, on la sèche sur sulfate de sodium et on la concentre. Le produit brut obtenu est purifié sur colonne de silice avec un mélange 1/1 d'acétate d'éthyle et d'heptane. On obtient 200 mg de produit.
Point de fusion : 118°C.

### Exemple 5 : 5-[4-(4,4,4-trifluorobutoxy)phényl]-3-(2-méthylthioéthyl)-1,3,4-oxadiazol-2(3H)-one

Dans un ballon tricol de 50 ml, on introduit 2 g de 5-[4-(4,4,4-trifluorobutoxy)phényl]-1,3,4-oxadiazol-2 (3H) -one, 1,14 g de 2-chloroéthyl-méthylsulfure, 2,4 g de carbonate de potassium et 30 ml de diméthylformamide. On chauffe le mélange réactionnel à 90°C et on le maintient à cette température pendant une nuit, puis on le verse dans de l'eau. On collecte le précipité formé par filtration, on le lave à l'eau et on le reprend par de l'acétate d'éthyle. On sèche la solution sur sulfate de sodium puis on évapore le solvant. Après recristallisation dans l'éther diisopropylique, on obtient 1,7 g de produit.
Point de fusion : 104,7°C.

Les composés de l'invention sont rassemblés dans le tableau suivant avec leurs caractéristiques physiques.

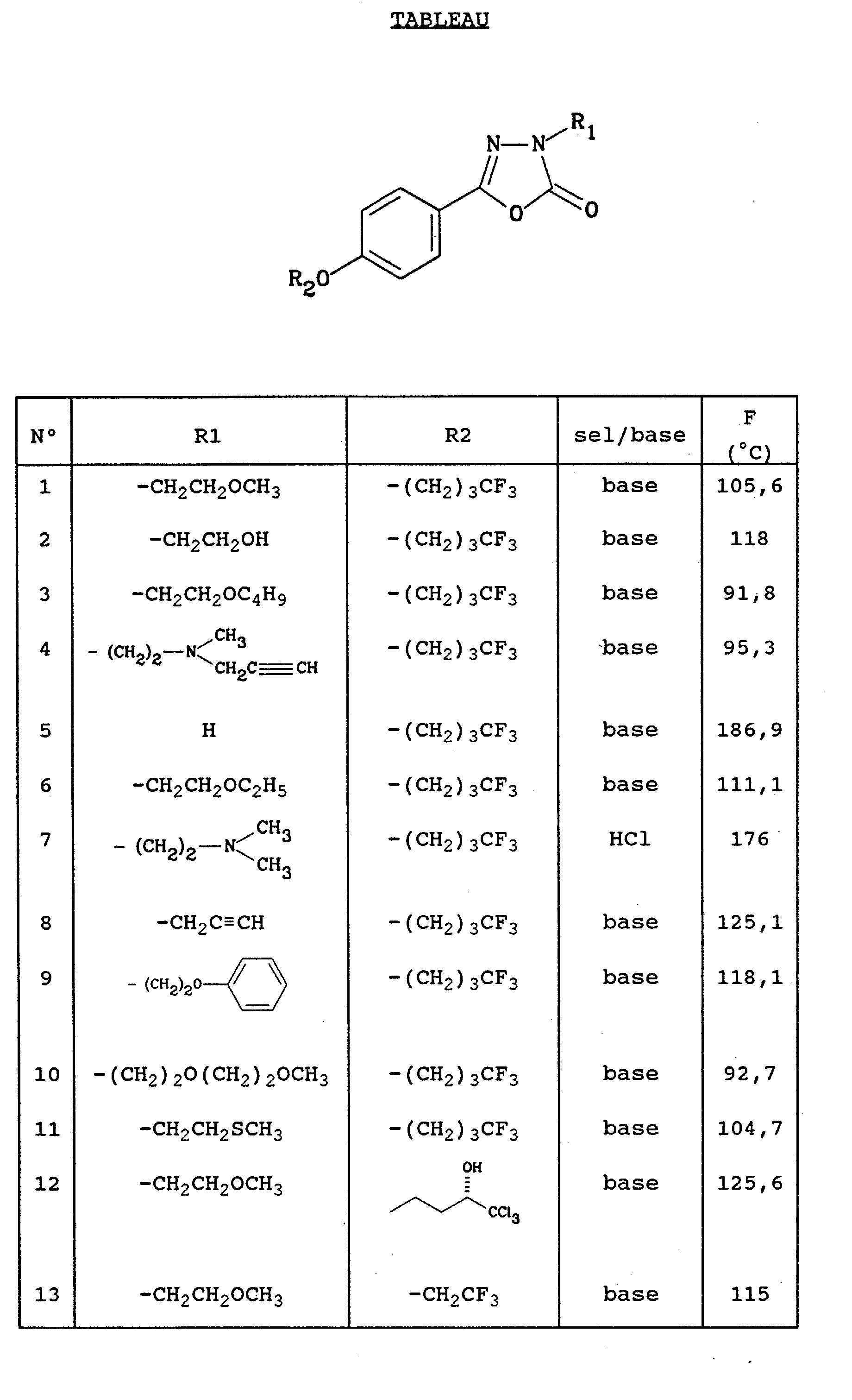

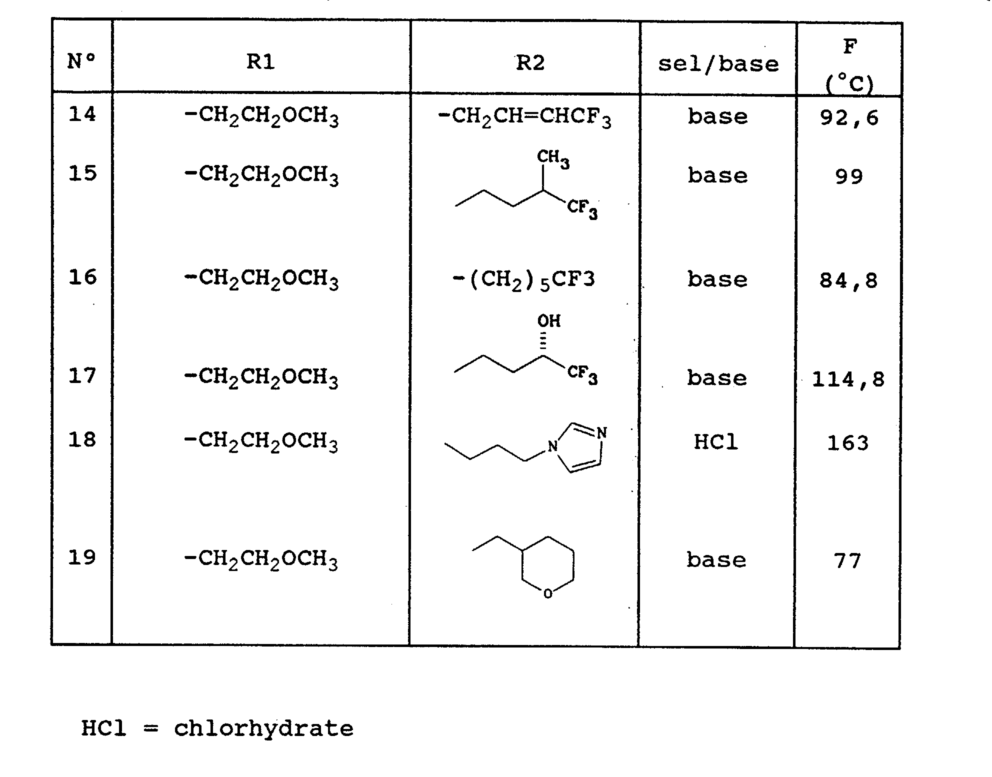

Les composés de l'invention ont fait l'objet d'essais pharmacologiques permettant de déterminer leur effet inhibiteur de la monoamine oxydase.
Ces essais consistent à mesurer l'activité in vitro de la monoamine oxydase-A (MAO-A) et de la monoamine oxydase-B (MAO-B).
Les mesures des activités MAO-A et MAO-B ont été réalisées en utilisant comme source d'enzyme un homogénat de cerveau de rat. La méthode utilisée, décrite par C. Fowler et M. Strolin-benedetti, dans J. Neurochem., 40, 1534-1541 (1983), consiste à préincuber l'enzyme 20 minutes à 37°C en présence ou en l'absence des inhibiteurs étudiés. Au terme de cette période, la réaction est démarrée par l'addition de [¹⁴C]sérotonine (5-HT, 125 µM), pour la mesure de l'activité de la MAO-A ou de [14C]phényléthylamine (PEA, 8 µM), pour la mesure de l'activité de la MAO-B. Après 5 minutes d'incubation à 37°C en présence de [¹⁴C]5-HT et 1 minute à 37°C en présence de [¹⁴C]PEA, la réaction est arrêtée par addition d'acide chlorhydrique. Les métabolites des amines sont alors extraits et la radioactivité est quantifiée par comptage en scintillation liquide.
L'activité inhibitrice vis à vis de la MAO-B est donnée par la constante d'inhibition Ki (MAO-B).
Pour les composés de l'invention, les Ki (MAO-B) varient entre 10⁻⁶ 6 et 10⁻⁹ M.
D'autre part, le rapport Ki(MAO-A)/ Ki(MAO-B) a été calculé.
Pour les composés de l'invention, ce rapport est compris entre 10³ et 10⁴.
Il apparaît donc que les composés de l'invention ont une activité inhibitrice de la MAO-B puissante et sélective.

Les composés de l'invention peuvent donc être utilisés pour la préparation de médicaments inhibiteurs de la monoamine oxydase B, ces médicaments trouvant leur emploi en thérapeutique notamment dans le traitement des troubles neurologiques liés au vieillissement pathologique, des troubles mnésiques, des troubles de l'humeur, de la schizophrénie, de la psychasthénie, du ralentissement psychique lié au vieillissement, de certaines formes de dépression et de la maladie de Parkinson.

Les composés de l'invention peuvent être présentés, en association avec des excipients, sous forme de compositions formulées en vue de l'administration par voie orale, parentérale ou rectale, par exemple sous forme de comprimés, dragées, capsules, solutions, suspensions ou suppositoires.

Par voie orale, la dose de principe actif administrée par jour peut atteindre 50 mg/kg, en une ou plusieurs prises. Par voie parentérale, elle peut atteindre 5 mg/kg et par voie rectale 10 mg/kg.

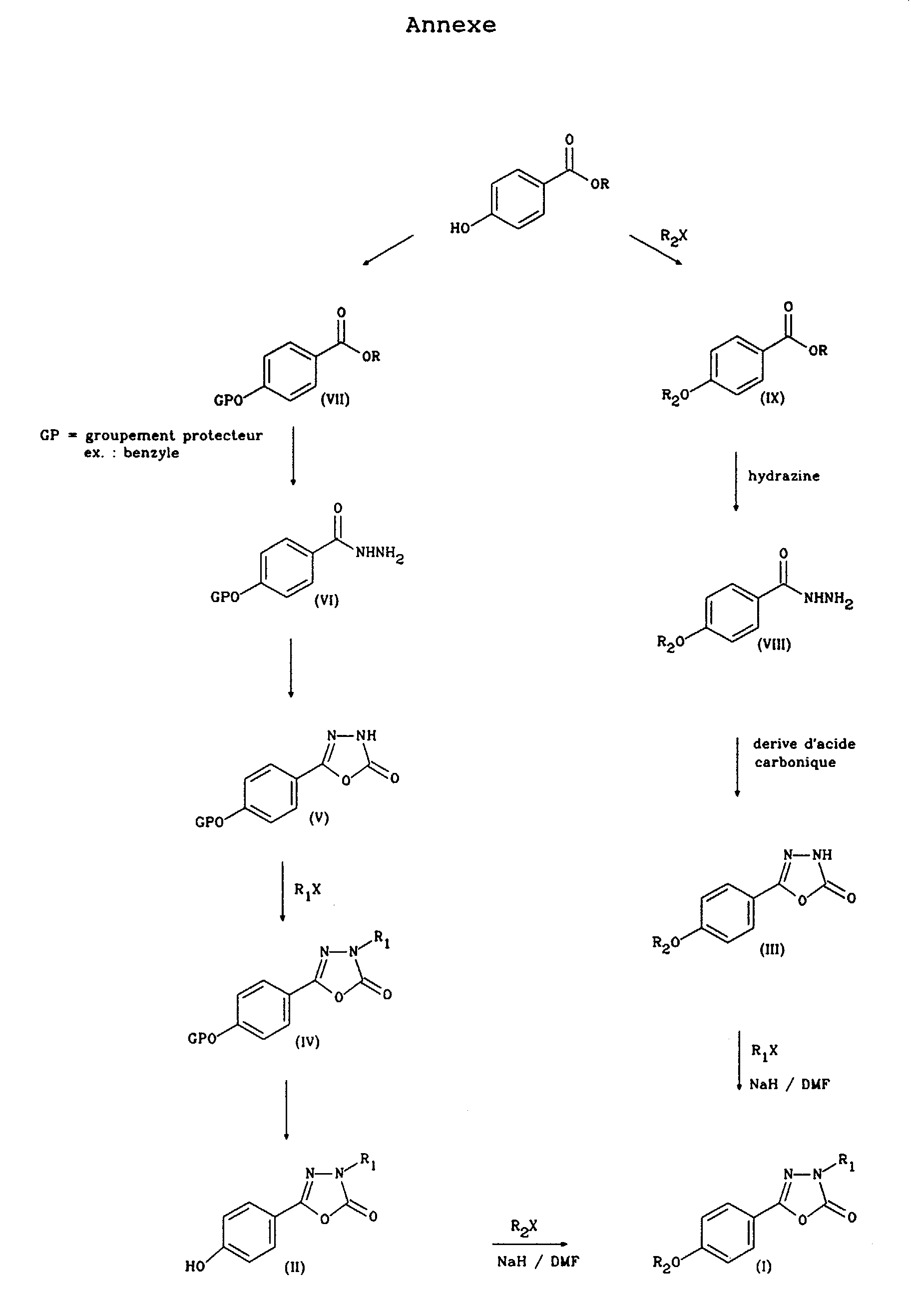

## Revendications

1. Dérivés de 1,3,4-oxadiazol-2(3H)-one, de formule générale (I) 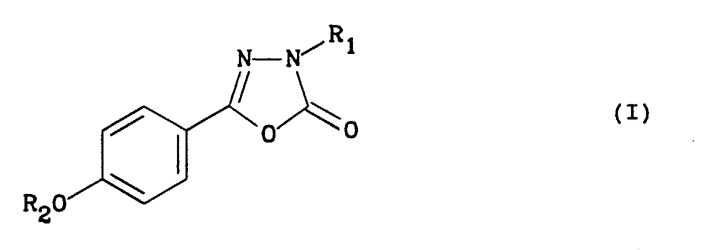 dans laquelle
R₁ représente soit un groupe (C₁₋₄)alkyle linéaire ou ramifié, substitué par un groupe hydroxy, phénoxy, (C₁₋₄)alcoxy, (C₁₋₄)alkylthio, mercapto,
(C₁₋₄)alcoxy-(C₁₋₄)alcoxy, di(C₁₋₄)alkylamino ou N-(C₁₋₄)alkyl-N-propynylamino, soit un groupe (C₃₋₄)alcynyle, et
R₂ représente soit un groupe (C₁₋₈)alkyle linéaire ou ramifié, substitué par un ou plusieurs atomes d'halogène et/ou un groupe hydroxy, 1-imidazolyle ou 3-tétrahydropyranyle, soit un groupe trifluoro(C₃₋₅)alcényle,
sous forme d'énantiomères purs ou de mélanges d'énantiomères, y compris de mélanges racémiques, ainsi que leurs sels d'addition aux acides pharmaceutiquement acceptables.

2. Le 5-[4-(4,4,4-trifluorobutoxy)phényl]-3-(2-méthoxyéthyl)-1,3,4-oxadiazol-2(3H)-one.

3. Le 5-[4-(4,4,4-trifluorobutoxy)phényl]-3-(2-hydroxyéthyl)-1,3,4-oxadiazol-2(3H)-one.

4. Le 5-[4-(4,4,4-trifluorobutoxy)phényl]-3-(2-méthylthioéthyl)-1,3,4-oxadiazol-2(3H)-one.

5. Le 5-[4-(4,4,4-trifluoro-2-butènyloxy)phényl]-3-(2-méthoxyéthyl)-1,3,4-oxadiazol-2(3H)-one.

6. Le 5-[4-(4,4,4-trifluoro-3(R)-hydroxybutoxy)phényl]-3-(2-méthoxyéthyl)-1,3,4-oxadiazol-2(3H)-one.

7. Le 5-[4-(tétrahydropyran-3-ylméthoxy)phényl]-3-(2-méthoxyéthyl)-1,3,4-oxadiazol-2(3H)-one.

8. Procédé de préparation des composés de formule (I) selon la revendication 1, qui consiste à traiter un composé de formule (II) 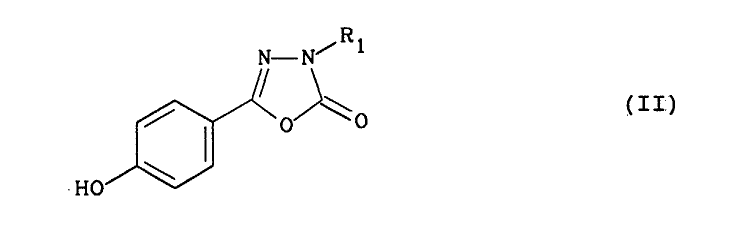 dans laquelle R₁ est défini comme dans la revendication 1, par un composé de formule R₂X, dans laquelle R₂ est défini comme dans la revendication 1 et X représente un atome d'halogène, le groupe mésyloxy ou tosyloxy, en présence d'une base telle que le carbonate de potassium ou l'hydrure de sodium, dans un solvant tel que l'acétonitrile ou le diméthylformamide.

9. Procédé de préparation des composés de formule (I) selon la revendication 1, qui consiste à traiter un composé de formule (III) 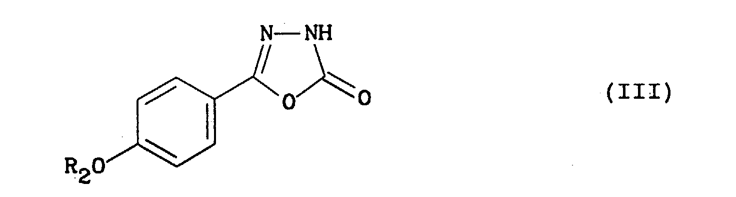 dans laquelle R₂ est défini comme dans la revendication 1, par un composé de formule R₁X, dans laquelle R₁ est défini comme dans la revendication 1 et X représente un atome d'halogène, le groupe mésyloxy ou tosyloxy, en présence d'une base telle que le carbonate de potassium ou l'hydrure de sodium, dans un solvant tel que le diméthylformamide.

10. Médicament caractérisé en ce qu'il consiste en un composé de formule (I) selon la revendication 1.

11. Composition pharmaceutique caractérisée en ce qu'elle contient un composé de formule (I) selon la revendication 1, en association avec tout excipient approprié.

## Patentansprüche

1.3.4-Oxadiazol-2(3H)-on-Derivate der allgemeinen Formel (I) 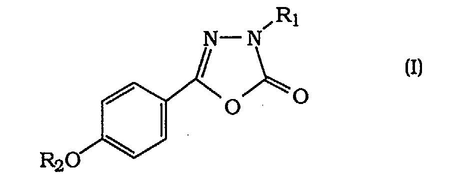 in der
R₁ entweder eine geradkettige oder verzweigte (C₁₋₄)-Alkylgruppe, die durch eine Hydroxylgruppe. Phenoxygruppe, (C₁₋₄)-Alkoxygruppe, (C₁₋₄)-Alkylthiogruppe, Mercaptogruppe, (C₁₋₄)-Alkoxy-(C₁₋₄)-alkoxygruppe, Di-(C₁₋₄)-alkylaminogruppe oder N-(C₁₋₄)-Alkyl-N-propinylaminogruppe substituiert ist, oder eine (C₃₋₄)-Alkinylgruppe und
R₂ entweder eine geradkettige oder verzweigte (C₁₋₈)-Alkylgruppe, die durch eines oder mehrere Halogenatome und/oder eine Hydroxylgruppe, 1-Imidazolylgruppe oder 3-Tetrahydropyranylgruppe substituiert ist, oder eine Trifluor-(C₃₋₅)-alkenylgruppe bedeuten,
in Form der reinen Enantiomeren oder Mischungen der Enantiomeren einschließlich racemischer Mischungen, sowie deren Additionssalze mit pharmazeutisch annehmbaren Säuren.

2. 5-[4-(4,4,4-Trifluorbutoxy)-phenyl]-3-(2-methoxyethyl)-1,3,4-oxadiazol-2(3H)-on.

3. 5- [4-(4,4,4-Trifluorbutoxy)-phenyl]-3-(2-hydroxyethyl)-1,3,4-oxadiazol-2(3H)-on.

4. 5-[4-(4,4,4-Trifluorbutoxy)-phenyl]-3-(2-methylthioethyl)-1,3,4-oxadiazol-2(3H)-on.

5. 5-[4-(4,4,4-Trifluor-2-butenyloxy)-phenyl]-3-(2-methoxyethyl)-1,3,4-oxadiazol-2(3H)-on.

6. 5-[4-(4,4,4-Trifluor-3(R)-hydroxybutoxy)-phenyl]-3-(2-methoxyethyl)-1,3,4-oxadiazol-2(3H)-on.

7. 5-[4-(Tetrahydropyran-3-yl-methoxy)-phenyl]-3-(2-methoxyethyl)-1,3,4-oxadiazol-2(3H)-on.

8. Verfahren zur Herstellung der Verbindungen der Formel (I) nach Anspruch 1, welches darin besteht, eine Verbindung der Formel (II) 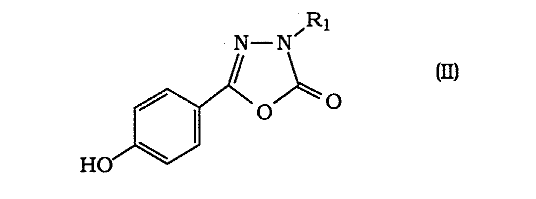 in der R₁ die in Anspruch 1 angegebenen Bedeutungen besitzt, mit einer Verbindung der Formel R₂X, in der R₂ die in Anspruch 1 angegebenen Bedeutungen besitzt und X für ein Halogenatom, eine Mesyloxygruppe oder eine Tosyloxygruppe steht, in Gegenwart einer Base, wie Kaliumcarbonat oder Natriumhydrid in einem Lösungsmittel, wie Acetonitril oder Dimethylformamid, zu behandeln.

9. Verfahren zur Herstellung der Verbindungen der Formel (I) nach Anspruch 1, welches darin besteht, eine Verbindung der Formel (III) 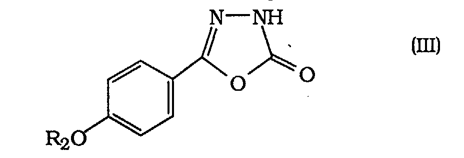 in der R₂ die in Anspruch 1 angegebenen Bedeutungen besitzt, mit einer Verbindung der Formel R₁X, in der R₁ die in Anspruch 1 angegebenen Bedeutungen besitzt und X ein Halogenatom, eine Mesyloxygruppe oder eine Tosyloxygruppe bedeutet, in Gegenwart einer Base, wie Kaliumcarbonat oder Natriumhydrid, in einem Lösungsmittel, wie Dimethylformamid, zu behandeln.

10. Arzneimittel, dadurch gekennzeichnet, daß es aus einer Verbindung der Formel (I) nach Anspruch 1 besteht.

11. Pharmazeutische Zubereitung, dadurch gekennzeichnet, daß sie eine Verbindung der Formel (I) nach Anspruch 1 in Kombination mit irgendeinem geeigneten Trägermaterial enthält.

## Claims

1. 1,3,4-Oxadiazol-2(3H)-one derivatives, of general formula (I) 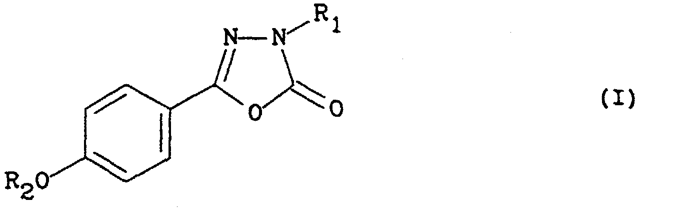 in which
R₁ represents either a linear or branched (C₁₋₄)alkyl group which is substituted with a hydroxyl group, a phenoxy group, a (C₁₋₄)alkoxy group, a (C₁₋₄)alkylthio group, a mercapto group, a (C₁₋₄)alkoxy-(C₁₋₄)alkoxy group, a di(C₁₋₄)alkylamino group or an N-(C₁₋₄)alkyl-N-propynylamino group, or represents a (C₃₋₄)alkynyl group, and
R₂ either represents a linear or branched (C₁₋₈)alkyl group which is substituted with one or more halogen atoms and/or a hydroxyl group, a 1-imidazolyl group or a 3-tetrahydropyranyl group, or represents a trifluoro-(C₃₋₅)alkenyl group,
in the form of pure enantiomers or mixtures of enantiomers, including racemic mixtures, as well as the addition salts thereof with pharmaceutically acceptable acids.

2. 5-[4-(4,4,4-Trifluorobutoxy)phenyl]-3-(2-methoxyethyl)-1,3,4-oxadiazol-2(3H)-one.

3. 5-[4-(4,4,4-Trifluorobutoxy)phenyl]-3-(2-hydroxyethyl)-1,3,4-oxadiazol-2(3H)-one.

4. 5-[4-(4,4,4-Trifluorobutoxy)phenyl]-3-(2-methylthioethyl)-1,3,4-oxadiazol-2(3H)-one.

5. 5-[4-(4,4,4-Trifluoro-2-butenyloxy)-phenyl]-3-(2-methoxyethyl)-1,3,4-oxadiazol-2(3H)-one.

6. 5-[4-(4,4,4-Trifluoro-3(R)-hydroxybutoxy)-phenyl]-3-(2-methoxyethyl)-1,3,4-oxadiazol-2(3H)-one.

7. 5-[4-(Tetrahydropyran-3-ylmethoxy)phenyl]-3-(2-methoxyethyl)-1,3,4-oxadiazol-2(3H)-one.

8. Process for preparing the compounds of formula (I) according to Claim 1, which consists in treating a compound of formula (II) 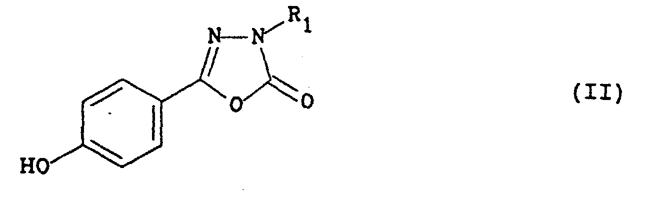 in which R₁ is defined as in Claim 1, with a compound of formula R₂X, in which R₂ is defined as in Claim 1 and X represents a halogen atom or the mesyloxy or tosyloxy group, in the presence of a base such as potassium carbonate or sodium hydride, in a solvent such as acetonitrile or dimethylformamide.

9. Process for preparing compounds of formula (I) according to Claim 1, which consists in treating a compound of formula (III) 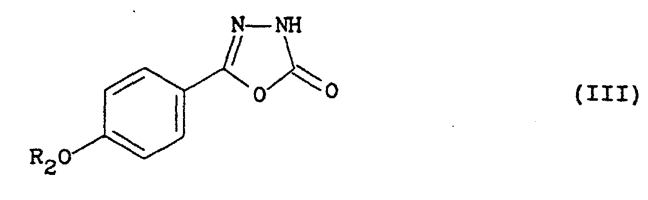 in which R₂ is defined as in Claim 1, with a compound of formula R₁X, in which R₁ is defined as in Claim 1 and X represents a halogen atom or the mesyloxy or tosyloxy group, in the presence of a base such as potassium carbonate or sodium hydride, in a solvent such as dimethylformamide.

10. Medicinal product, characterized in that it consists of a compound of formula (I) according to Claim 1.

11. Pharmaceutical composition, characterized in that it contains a compound of formula (I) according to Claim 1, in combination with any appropriate excipient.
